# EUROPÄISCHE PATENTANMELDUNG

(11) **EP 1 061 478 A2**
(43) Veröffentlichungstag der Anmeldung: **20.12.2000**
(21) Anmeldenummer: 00111916.3
(22) Anmeldetag: 14.06.2000
(51) Int. Cl.: G06T 15/70

(54) **Vorrichtung und Verfahren zur Animation einer Person**

(30) Priorität: 14.06.1999 DE 29910326 U
(71) Anmelder: Maier, Folker, 74538 Rosengarten-Westheim (DE)
(72) Erfinder: Maier, Folker, 74538 Rosengarten-Westheim (DE)
(74) Vertreter: Kitzhofer, Thomas, Dipl.-Ing.

(57) **Zusammenfassung**

Eine Vorrichtung zur Animation einer Person ist mit wenigstens einem optischen Sensor (5), wenigstens einem die Bewegungen der Vorrichtung oder der zu animierenden Person aufnehmenden Bewegungssensor (7), wenigstens einem akustischen Sensor (9), wenigstens einer Auswertelektronik (13) und wenigstens einer Anzeige (15, 19, 21) ausgestattet, wobei die Sensoren (3) und die Anzeige (15, 19, 21) mit der Auswertelektronik (13) verbunden sind und die Auswertelektronik (13) so ausgebildet ist, daß sie in Abhängigkeit von den von den Sensoren (3) erfaßten Energiemengen ein von der zu animierenden Person wahrnehmbares Signal über die Anzeige (15, 19, 21) abgeben kann. Ferner ist ein entsprechend arbeitendes Verfahren zur Animation einer Person beschrieben.

## Beschreibung

Die Erfindung betrifft eine Vorrichtung zur Animation einer Person, insbesondere eine tragbare, mobile Vorrichtung.

Auf den menschlichen Körper wirken verschiedene Energieformen, z.B. Licht, akustische Energie oder andere Energiearten oder Energiefelder (elektrische, magnetische oder elektromagnetische Felder) ein. Der Mensch ist jedoch nicht nur den von außen auf ihn einwirkenden Energien ausgesetzt, die seinen Gesundheitszustand und sein Wohlbefinden beeinflussen, sondern auch äußeren Einflüssen wie der Luft, die er einatmet, und deren Zusammensetzung. Er kann auch durch eigene Aktivität auf seinen Gesundheitszustand einwirken, denn die Gesundheit und das menschliche Wohlbefinden werden ferner auch durch den Umfang der körperlichen Betätigung beeinflußt. Am vorteilhaftesten ist es, wenn ein bestimmtes Verhältnis von Umgebungsbedingungen und körperlicher Betätigung vorhanden ist. Beispielsweise sollte man nicht zuviel aber auch nicht zuwenig Sonne oder, allgemein, Licht ausgesetzt sein und sich nicht zuwenig, aber auch nicht unbedingt zuviel bewegen. Jeder Mensch hat ein anderes für ihn optimales Verhältnis der auf ihn einwirkenden Energiemengen der unterschiedlichen Energieformen zum Ausmaß von körperlicher Bewegung. Obwohl man selbst weiß, daß man dieses optimale Verhältnis nicht erreicht, fehlt es oft an Anreizen, sich z.B. mehr zu bewegen oder sich aktiv und bewußt mehr oder weniger Sonne auszusetzen, und es fehlt auch an einer halbwegs quantitativen Angabe über die Umgebungsbedingungen, z.B. in Form der auf den Körper einwirkenden Energiemengen und -formen, sowie über die körperliche Betätigung. Es gibt zwar beispielsweise einen tragbaren Schrittmesser für Läufer, doch ist dieser nicht geeignet, während des normalen Alltages seinem Träger eine Angabe über den Umfang der erfolgten körperlichen Betätigung zu machen.

Die Erfindung schafft eine Vorrichtung, die der Person eine Information über wenigstens einen Teil der auf sie einwirkenden Umgebungsenergie und den Umfang ihrer körperlichen Betätigung geben kann, um sie zu animieren, das geplante Verhalten zu ändern.

Dies wird bei einer Vorrichtung der eingangs genannten Art dadurch erreicht, daß die Vorrichtung mit wenigstens einem optischen Sensor, wenigstens einem die Bewegungen der Vorrichtung oder der zu animierenden Person aufnehmenden Bewegungssensor, wenigstens einem akustischen Sensor, wenigstens einer Auswertelektronik und wenigstens einer Anzeige ausgestattet ist. Die Sensoren und die Anzeige sind mit der Auswertelektronik verbunden, und die Auswertelektronik ist derart ausgebildet, daß sie in Abhängigkeit von den von den Sensoren erfaßten Energiemengen ein von der zu animierenden Person wahrnehmbares Signal über die Anzeige abgeben kann. Die erfindungsgemäße Vorrichtung erfaßt wenigstens die auf den Träger der Vorrichtung einwirkende Lichtmenge sowie den auf ihn einwirkenden Schall. Darüber hinaus wird über einen Bewegungssensor die kinetische Energie der Vorrichtung, falls diese als mobile Vorrichtung am Körper getragen wird und damit auch eine Aussage über die körperliche Betätigung gibt, erfaßt. Sämtliche Energiemengen werden zur Auswertelektronik geleitet und von dieser verarbeitet. Die Auswertelektronik steuert das von der Anzeige abgegebene Signal.

Bei Untersuchungen hat sich herausgestellt, daß die drei Energieformen Licht, Bewegung und Schall sehr wichtig für den Gesamtzustand eines Menschen sind. Diese drei Energieformen werden von der erfindungsgemäßen Vorrichtung erfaßt und verarbeitet. Der Träger der Vorrichtung erhält über das Signal vorzugsweise eine Aussage über wenigstens einen Teil der auf ihn einwirkenden Umgebung und zusätzlich auch über seine körperliche Betätigung. Darauf abgestimmt erhält er über das Signal einen Anreiz, sich anders zu verhalten, sich z.B. in einem anderen Umfang körperlich zu betätigen oder sich gegebenenfalls einer anderen Umgebung auszusetzen, sich z.B. mehr oder weniger Licht oder Schall auszusetzen.

Zusätzlich kann wenigstens einer der folgenden Sensoren vorhanden und mit der Auswertelektronik verbunden sein: ein Temperatursensor, ein elektrische, magnetische oder elektromagnetische Felder erfassender Sensor oder ein Sensor zum Bestimmen von Gasen. Durch noch mehr Sensoren können noch bessere Aussagen über die Umgebung des Trägers der Vorrichtung gemacht werden.

Die Anzeige ist eine optische und/oder akustische Anzeige, z.B. eine LED- oder LCD-Anzeige.

Die erfindungsgemäße Vorrichtung hat ferner nach der bevorzugten Ausführungsform einen mit der Auswertelektronik gekoppelten Zeitmesser. Dieser ermöglicht es, daß auch die Zeit in die Messung und Verarbeitung der Signale mit eingeht. Beispielsweise können während bestimmter Zeiträume über die Sensoren die Energiemengen gemessen werden, oder die Auswertelektronik wird nur in vorbestimmten Zeiträumen die erfaßten Energiemengen aufnehmen und verarbeiten. Ferner kann sich über den Zeitmesser die Vorrichtung an jedem Tag neu zurücksetzen, oder es können über mehrere Tage oder nur über Stunden hinweg die Energiemengen erfaßt werden.

Die erfindungsgemäße Vorrichtung kann die erfaßten Energiemengen permanent anzeigen, was auch umfaßt, jederzeit auf Knopfdruck die Energiemengen anzuzeigen. Es muß im übrigen nicht unbedingt jede Energiemenge in physikalischen Einheiten angegeben werden, es reicht auch eine beliebig skalierte Anzeige oder eine Ein-Aus-Anzeige. Darüber hinaus muß nicht die Energiemenge jeder Energieform angegeben werden, vielmehr ist das Verhältnis der von den einzelnen Sensoren erfaßten Energiemenge untereinander wichtiger, so daß das Verhältnis der Energiemengen angezeigt wird. Dabei kann permanent das Verhältnis der erfaßten Energiemengen angegeben werden oder auch nur diejenige Energieform, die zuviel oder zuwenig auf den Träger eingewirkt hat. Ferner kann die Anzeige nur bei Erreichen einer vorbestimmten Energiemenge oder eines vorbestimmten Verhältnisses der Energiemengen zueinander ein Signal abgeben. Beispielsweise wird jedesmal dann ein Signal abgegeben, wenn der Träger sich genügend bewegt hat, wenn er genügend Licht und wenn er genügend akustische Reize erhalten hat. Oder es kann dann ein Signal erfolgen, wenn ein gewünschtes oder nicht gewünschtes Verhältnis der von den einzelnen Sensoren erfaßten Energiemengen auftritt.

Vorzugsweise ist die Auswertelektronik mit einem von außen programmierbaren Datenspeicher verbunden, in dem die vorbestimmten Energiemengen oder die Verhältnisse der Energiemengen angegeben sind, ab deren Erreichen ein Signal erzeugt werden soll. Der Träger oder sein Therapeut können damit die Vorrichtung auf den Träger selbst und seine Bedürfnisse maßschneidern.

Die erfindungsgemäße Vorichtung weist vorzugsweise eine eigene Energiequelle auf, z.B. eine Batterie oder einen Akkumulator.

Die erfindungsgemäße Vorrichtung ist vorzugsweise als am Körper oder der Kleidung zu tragendes Gerät, z.B. Schmuckstück, ausgebildet. Bei der Ausbildung als Schmuckstück ist es auch im Alltag tragbar und erweckt nicht den Eindruck, ein medizinisches Gerät zu sein.

Gemäß einer weiteren Ausgestaltung ist die Vorrichtung mehrteilig ausgebildet und besteht aus einem, am Körper zu tragenden Gerät sowie einem Auswertegerät, z.B. einem Laptop, an den das zu tragende Gerät wahlweise ankoppelbar ist und das die vom Gerät aufgenommenen Daten analysiert. Das Auswertegerät kann die gesamte Auswertung übernehmen und die Signale abgeben oder es kann eine über die im mobilen Gerät vorgesehene hinausgehende weitergehende Analyse vornehmen.

Eine Ausgestaltung der Erfindung sieht vor, daß die Sensoren in einer herkömmlichen Videokamera, vorzugsweise einer digitalen Videokamera untergebracht sind. Ein mit der Videokamera verbundener Computer ist als Auswertelektronik vorgesehen. Eine solche Ausführungsform könnte beispielsweise im Büro der zu animierenden Person untergebracht sein. Die Person wird den ganzen Tag oder nur zeitweise, ebenso wie die unmittelbare Umgebung der Person beobachtet. Die Energiemengen werden ermittelt, und, abhängig von diesen, wird das entsprechende Signal an die Person abgegeben. Die Person kann zu einem bewußteren Handeln oder Leben "erzogen" werden.

Weitere Faktoren, welche aber nicht von außen ermittelt werden können, beispielsweise psychische Faktoren wie der Gemütszustand der Person, können die Vorrichtung gemäß einer besonderen Ausgestaltung auch beeinflussen. Hierzu ist vorgesehen, daß in der Auswerteelektronik Fragelisten abgespeichert sind. Die Auswertelektronik stellt über die Anzeige der Person Fragen, z.B. das subjektive Empfinden über die Leistungsfähigkeit, oder animiert die Person, dem Gerät Fragen zu stellen. Über ein Eingabegerät kann die Person die Fragen beantworten oder Fragen stellen, wobei die Eingabe die spätere Anzeige mitbeeinflußt. Es hat beispielsweise keinen Sinn, die Person zu starker körperlicher Betätigung zu animieren, wenn sich die Person gerade körperlich sehr wenig leistungsfähig einschätzt. Ferner könnte beispielsweise die Person dem Gerät die Frage stellen, welche Abendbeschäftigung es vorschlägt, und das Gerät würde, abhängig von den Daten, die es aufgenommen hat, z.B. eine überwiegend durch körperliche Betätigung geprägte Abendbeschäftigung im Freien oder z.B. Entspannen im Dunklen vorschlagen. Auch die Art, die Eingabe zu machen (z.B. schnelles Eintippen, ruckartiges oder zittriges Bewegen mit der Maus), kann vom Gerät erfaßt und ausgewertet werden und Auswirkungen auf die Ausgabe haben.

Die Erfindung betrifft darüber hinaus ein Verfahren zum Animieren einer Person, das durch folgende Schritte gekennzeichnet ist:
a) die Lichtmenge, die auf die Person einwirkt, wird durch eine Vorrichtung ermittelt,
b) der Lärm, dem die Person ausgesetzt ist und/oder den die Person erzeugt, wird durch die Vorrichtung ermittelt,
c) Bewegungen der Person werden durch die Vorrichtung ermittelt,
d) die in den Schritten a) bis c) ermittelten Daten werden zu einer Auswertelektronik gesandt und von dieser ausgewertet,
e) abhängig von den ermittelten Daten erzeugt die Auswertelektronik ein von der Person wahrnehmbares Signal oder eine schriftliche Information.

Gemäß der bevorzugten Ausführungsform des Verfahrens sind in der Auswertelektronik vorbestimmte Fragen zur Person abgespeichert, die über die Anzeige der Person gestellt werden. Eine Eingabe wird der Person zur Verfügung gestellt, die mit der Auswertelektronik verbunden ist und über die die Person Angaben zur Beantwortung der Fragen geben kann. Abhängig von den Signalen und von der Eingabe wird in der Auswertelektronik ein Signal erzeugt.

Weitere Merkmale und Vorteile der Erfindung ergeben sich aus der nachfolgenden Beschreibung und aus den nachfolgenden Zeichnungen, auf die Bezug genommen wird. In den Zeichnungen zeigen:
Fig. 1 eine Frontansicht einer vereinfacht dargestellten Ausführungsform der erfindungsgemäßen Vorrichtung, von der das erfindungsgemäße Verfahren durchgeführt wird,
Fig. 2 eine Längsschnittansicht durch die Vorrichtung nach Fig. 1 und
Fig. 3 eine Schemadarstellung, die den Aufbau der erfindungsgemäßen Vorrichtung zeigt.

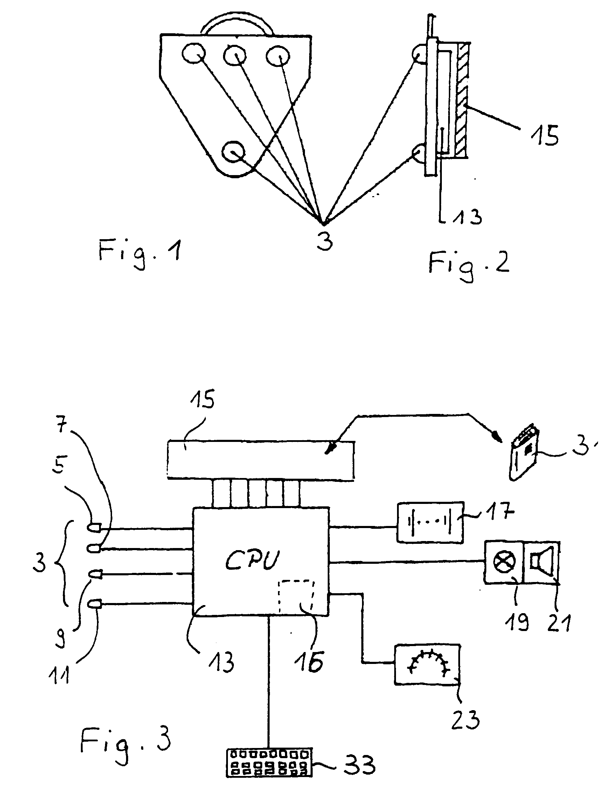

In Fig. 1 ist eine an der Kleidung zu befestigende Vorrichtung dargestellt, die auf ihrer Vorderseite mehrere Sensoren 3 aufweist. Diese Sensoren sind auch in Fig. 3 zu erkennen. Dabei handelt es sich um einen optischen Sensor 5 (z.B. Foto-Frequenzwandler), einen Bewegungssensor 7 (z.B. einen Neigungsschalter), der die Bewegung der Vorrichtung und damit die kinetische Energie erfaßt, einen akustischen Sensor 9, der den Umfang der Beschallung der Vorrichtung ermittelt, und einen Sensor 11, der exemplarisch für einen oder mehrere der folgenden Sensoren steht: einen Temperatursensor, einen ein elektrisches Feld (auf Vorhandensein und/oder die Feldstärke) bestimmenden Sensor, einen ein magnetisches Feld (auf Vorhandensein und/oder die Feldstärke) bestimmenden Sensor, einen ein elektromagnetisches Feld (auf Vorhandensein und/oder die Feldstärke) bestimmenden Sensor und einen ein bestimmtes oder mehrere Gase bestimmenden Sensor (z.B. CO₂-Gehalt der Luft).

Die Sensoren 3 sind mit einer Auswertelektronik 13 in Form einer CPU gekoppelt. Mit der Auswertelektronik 13 ist auch eine LCD-Anzeige 15 an der Rückseite der Vorrichtung gekoppelt. Darüber hinaus weist die gezeigte Vorrichtung eine eigene Energiequelle in Form einer Batterie 17 sowie eine LED-Anzeige 19 und eine akustische Anzeige 21, auch akustischer Signalgeber genannt, und schließlich einen Zeitmesser 23 auf. Sämtliche Anzeigen, die Energiequelle und der Zeitmesser sind ebenfalls mit der Auswertelektronik 13 verbunden. In der Auswertelektronik ist ferner ein Datenspeicher 16 integriert, der über nicht gezeigte Anschlüsse von außen programmierbar ist, beispielsweise über einen PC.

Die Funktionsweise der erfindungsgemäßen Vorrichtung wird im folgenden erläutert. Die äußerlich als Brosche gestaltete Vorrichtung erfaßt über die Sensoren 5 bis 11 die auf die Vorrichtung treffende Lichtmenge, die auf die Vorrichtung treffende Schallenergie, die kinetische Energie, die die Bewegung der Vorrichtung und damit das Ausmaß der körperlichen Bewegung der die Vorrichtung tragenden Person wiedergibt, und z.B. die Umgebungstemperatur. Die Vorrichtung setzt sich jeden Tag auf die Ausgangsstellung zurück, nullt sich sozusagen täglich, was deshalb selbständig erfolgen kann, da der Zeitmesser 23 sowohl eine Kalenderfunktion als auch einen Tageszeitmesser beinhaltet.

Im Datenspeicher 16 ist ein bestimmtes Verhältnis der Mengen unterschiedlicher Energieformen, die von den Sensoren 3 erfaßt werden, abgespeichert. Sobald dieses Verhältnis während des Tages erreicht wird, werden über die Anzeigen 15, 19, 21 Signale abgegeben. Ein akustisches Signal sowie ein Leuchtsignal sollen den Träger der Vorrichtung veranlassen, auf die LCD-Anzeige 15 zu sehen. Auf dieser Anzeige wird ein Buchstaben- und/oder Nummerncode sichtbar oder, wenn die Anzeige größer ausgebildet sein kann, eine schriftliche Mitteilung erscheinen. Bei Code-Anzeigen kann ein Buch 31 darüber Auskunft geben, was dieser Code bedeutet oder wie sich der Träger der Vorrichtung verhalten soll, wenn das Signal ertönt und erscheint und die Anzeige Informationen gibt.

Es können im Datenspeicher 16 auch mehrere vorgegebene Werte angegeben sein, bei deren Erreichen die Auswertelektronik 13 jeweils ein Signal initiiert. Auch ein Eingabegerät 33, z.B. eine ansteckbare Tastatur, kann vorgesehen sein, über die die Person Eingaben machen kann, wie später noch erläutert wird.

Bei verschiedenen Verhältnissen der Energiemengen können ferner unterschiedliche Signale und unterschiedliche Anzeigen initiiert werden. Darüber hinaus ist es möglich, daß die Vorrichtung gemäß einer anderen Ausführungsform auch die Menge an Energie, die von den Sensoren 3 erfaßt wird, anzeigt, was in einer permanenten Anzeige erfolgen kann. Damit wird der Träger der Vorrichtung u.a. darauf hingewiesen, sich mehr zu bewegen. Ferner kann beispielsweise für jede Energieform ein Grenzwert vorgegeben werden, den der Träger der Vorrichtung während des Tages erreichen soll, wobei bei Erreichen jedes Grenzwertes einer Energieform ein optisches und akustisches Signal ertönt.

Die Ausführungsform nach den Fig. 1 bis 3 kann auch so ausgebildet sein, daß die Vorrichtung zweiteilig ausgebildet ist, indem nämlich ein am Körper zu tragendes Gerät, z.B. das in Fig. 1 gezeigte Schmuckstück, vorgesehen ist, welches aber die Auswertelektronik nicht oder nur teilweise enthält. Ein wahlweise mit dem am Körper zu tragenden Gerät koppelbares Auswertegerät, z.B. ein Laptop, übernimmt dann die Auswertung komplett oder teilweise. Die Tastatur des Laptops bildet ein Eingabegerät, über das die Person Eingaben in die Vorrichtung, hier das Auswertegerät, einspeisen kann. In dem Auswertegerät können Fragenkataloge abgespeichert sein, die über den Bildschirm oder eine andere Anzeige an die Person gerichtet werden. Wie bereits zuvor erwähnt, kann das von der Auswertelektronik abgegebene Signal abhängig von der Eingabe der Person sein. Vorzugsweise sind in der Auswertelektronik zahlreiche vorgegebene Signale mit zugeordneten Energiemengen oder Eingaben abgespeichert.

Eine nicht dargestellte Ausführungsform sieht vor, die Vorrichtung aus einer konventionellen Videokamera, die an einen Computer angeschlossen ist, zu bilden. Die Sensoren werden bei dieser Ausgestaltung durch das Mikrofon der Videokamera und deren Bildaufnehmer gebildet. Über den Bildaufnehmer und die Auswertelektronik wird nicht nur die Person, das heißt deren Bewegung, sondern auch die auf die Person einwirkende Umgebung (Licht, Bewegungen der Umwelt) erfaßt. Die Auswertelektronik sammelt die Daten, analysiert sie und gibt schließlich ein Signal über den Bildschirm ab, welches an die Person gerichtet ist.

## Patentansprüche

1. Vorrichtung zur Animation einer Person mit
wenigstens einem optischen Sensor (5),
wenigstens einem die Bewegungen der Vorrichtung oder der zu animierenden Person aufnehmenden Bewegungssensor (7),
wenigstens einem akustischen Sensor (9),
wenigstens einer Auswertelektronik (13) und
wenigstens einer Anzeige (15, 19, 21),
wobei die Sensoren (3) und die Anzeige (15, 19, 21) mit der Auswertelektronik (13) verbunden sind und
die Auswertelektronik (13) so ausgebildet ist, daß sie in Abhängigkeit von den von den Sensoren (3) erfaßten Energiemengen ein von der zu animierenden Person wahrnehmbares Signal über die Anzeige (15, 19, 21) abgeben kann.

2. Vorrichtung nach Anspruch 1, dadurch gekennzeichnet, daß zusätzlich wenigstens einer der folgenden Sensoren vorhanden und mit der Auswertelektronik (13) verbunden ist:
- ein Temperatursensor,
- ein ein elektrisches Feld bestimmender Sensor,
- ein ein magnetisches Feld bestimmender Sensor,
- ein ein elektromagnetisches Feld bestimmender Sensor und
- ein Sensor zum Bestimmen von Gasen.

3. Vorrichtung nach Anspruch 1 oder 2, dadurch gekennzeichnet, daß die Anzeige (15, 19, 21) eine optische und/oder akustische Anzeige ist.

4. Vorrichtung nach Anspruch 3, dadurch gekennzeichnet, daß die Anzeige (15, 19) eine LCD- oder eine LED-Anzeige ist.

5. Vorrichtung nach einem der vorhergehenden Ansprüche, gekennzeichnet durch einen mit der Auswertelektronik (13) gekoppelten Zeitmesser (23), wobei die Auswertelektronik (13) so ausgebildet ist, daß die Sensoren (3) nur in vorbestimmten Zeiträumen die von den Sensoren (3) abgegebenen Signale aufnimmt oder daß sie die Vorrichtung nur in bestimmten Zeiträumen aktiviert.

6. Vorrichtung nach einem der vorhergehenden Ansprüche, dadurch gekennzeichnet, daß eine permanente Anzeige vorhanden ist.

7. Vorrichtung nach einem der vorhergehenden Ansprüche, dadurch gekennzeichnet, daß die Vorrichtung die von den Sensoren (3) erfaßten Energiemengen anzeigt.

8. Vorrichtung nach Anspruch 6 oder 7, dadurch gekennzeichnet, daß sie nur bei Erreichen einer vorbestimmten Energiemenge, die von einem oder mehreren Sensoren (3) erfaßt wird, ein Signal abgibt.

9. Vorrichtung nach einem der vorhergehenden Ansprüche, dadurch gekennzeichnet, daß die Vorrichtung abhängig vom Verhältnis der von den einzelnen Sensoren erfaßten Energiemengen ein Signal abgibt.

10. Vorrichtung nach Anspruch 9, dadurch gekennzeichnet, daß die Anzeige (15, 19, 21) bei Erreichen eines vorbestimmten Verhältnisses der von den einzelnen Sensoren (3) erfaßten Energiemengen ein Signal abgibt.

11. Vorrichtung nach Anspruch 8 oder 10, dadurch gekennzeichnet, daß die Auswertelektronik (13) mit einem von außen umprogrammierbaren Datenspeicher (16) verbunden ist, in dem die vorbestimmten Energiemengen oder Verhältnisse der Mengen der verschiedenen Energiearten angegeben sind, bei deren Erreichen ein Signal abgegeben wird.

12. Vorrichtung nach einem der vorhergehenden Ansprüche, dadurch gekennzeichnet, daß die Vorrichtung eine eigene Energiequelle (17) aufweist.

13. Vorrichtung nach einem der vorhergehenden Ansprüche, dadurch gekennzeichnet, daß sie ein mobiles, am Körper der zu animierenden Person zu tragendes Gerät ist, welches die Sensoren aufweist.

14. Vorrichtung nach Anspruch 13, dadurch gekennzeichnet, daß die Sensoren in dem am Körper zu tragenden Gerät vorgesehen sind und daß ein Auswertegerät vorgesehen ist, an das das zu tragende Gerät wahlweise ankoppelbar ist und das die vom zu tragenden Gerät aufgenommenen Daten analysiert.

15. Vorrichtung nach einem der vorhergehenden Ansprüche, dadurch gekennzeichnet, daß die Sensoren Teil einer Videokamera sind und die Auswertelektronik ein mit der Videokamera verbundener, entsprechend programmierter Computer ist.

16. Vorrichtung nach einem der vorhergehenden Ansprüche, dadurch gekennzeichnet, daß ein Eingabegerät vorgesehen ist, über das die Person Eingaben in die Vorrichtung einspeisen kann.

17. Verfahren zum Animieren einer Person, gekennzeichnet durch folgende Schritte:
a) die Lichtenergie, die auf die Person einwirkt, wird durch eine Vorrichtung ermittelt,
b) der Lärm, dem die Person ausgesetzt ist und/oder den sie erzeugt, wird durch die Vorrichtung ermittelt,
c) Bewegungen der Person werden durch die Vorrichtung ermittelt,
d) die in den Schritten a) bis c) ermittelten Daten werden zu einer Auswertelektronik gesandt und von dieser ausgewertet,
e) abhängig von den ermittelten Daten erzeugt die Auswertelektronik ein von der Person wahrnehmbares Signal oder eine schriftliche Information.

18. Verfahren nach Anspruch 17, dadurch gekennzeichnet, daß in der Auswertelektronik vorbestimmte Fragen, die an die Person gerichtet werden, abgespeichert sind und daß über eine Anzeige der Person abgespeicherte Fragen gestellt werden, daß eine Eingabe für die Person zur Verfügung gestellt wird, die mit der Auswertelektronik verbunden ist und über die die Person Eingaben zur Beantwortung der Fragen geben kann, und daß das Signal von den ermittelten Daten und der Eingabe abhängt.

19. Verfahren nach Anspruch 17 oder 18, dadurch gekennzeichnet, daß das Verfahren unter Verwendung der Vorrichtung nach einem der Ansprüche 1 bis 16 durchgeführt wird.
